# EUROPEAN PATENT SPECIFICATION

(11) **EP 1 949 874 B1**
(45) Date of publication and mention of the grant of the patent: **12.08.2015**
(21) Application number: 08003235.2
(22) Date of filing: 27.10.2000
(51) Int. Cl.: A61F 2/958

(54) **End sleeve coating for stent delivery**
Endhülsenbeschichtung zur Stenteinbringung
Revêtement d'extrémité de manchon pour mise en place d'endoprothèse vasculaire

(30) Priority: 27.10.1999 US 427805
(43) Date of publication of application: 30.07.2008
(62) Divisional of application: 00989735.6
(73) Proprietor: Boston Scientific Limited, Hamilton HM11 (BM)
(72) Inventor: Wang, Lixiao, Maple Grove, MN 55369 (US); Tran, The Thomas, Coon Rapids,MN 55433 (US); Dicaprio, Fernando, St. Paul, MN 55105 (US); Williams, Brett A., Lino Lakes MN 55038 (US)
(74) Representative: Vossius & Partner Patentanwälte Rechtsanwälte mbB

(56) References cited:
- EP-A1- 0 688 545
- US-A- 5 295 978
- US-A- 5 944 726

## Description

### BACKGROUND OF THE INVENTION

The patent relates to a delivery system in which a catheter carries on its distal end portion a stent which is held in place around the catheter prior to and during percutaneous delivery by means of one and preferably two end sleeves which have been coated with a lubricious material. The lubricious material is added to the sleeve material subsequent to extrusion of the sleeve material but prior to a heat curing step. As a result of the heat curing the lubricious material attains a gel or jellied consistency. The stent may be self-expanding, such as a NITINOL shape memory stent, or it may be expandable by means of an expandable portion of the catheter, such as a balloon.

Stents and stent delivery systems are utilized in a number of medical procedures and situations, and as such their structure and function are well known. A stent is a generally cylindrical prosthesis introduced via a catheter into a lumen of a body vessel in a configuration having a generally reduced diameter and then expanded to the diameter of the vessel. In its expanded configuration, the stent supports and reinforces the vessel walls while maintaining the vessel in an open, unobstructed condition.

Both self-expanding and inflation expandable stents are well known and widely available in a variety of designs and configurations. Self-expanding stents must be maintained under a contained sheath or sleeve(s) in order to maintain their reduced diameter configuration during delivery of the stent to its deployment site. Inflation expandable stents are crimped to their reduced diameter about the delivery catheter, then maneuvered to the deployment site and expanded to the vessel diameter by fluid inflation of a balloon positioned between the stent and the delivery catheter. The present invention is particularly concerned with delivery and deployment of inflation expandable stents, although it is generally applicable to self-expanding stents when used with balloon catheters.

An example is the stent described in PCT Application NO. 960 3092 A1, published 8 February 1996.

In advancing an inflation expandable stent through a body vessel to the deployment site, there are a number of important considerations. The stent must be able to securely maintain its axial position on the delivery catheter, without translocating proximally or distally, and especially without becoming separated from the catheter. The stent, particularly any potentially sharp or jagged edges of its distal and proximal ends, must be protected to prevent edge dissection and prevent abrasion and/or reduce trauma of the vessel walls.

Inflation expandable stent delivery and deployment systems are known which utilize restraining means that overlie the stent during delivery. U. S. Patent No.4,950,227 to Savin et al., relates to an inflation expandable stent delivery system in which a sleeve overlaps the distal or proximal margin (or both) of the stent during delivery. During inflation of the stent at the deployment site, the stent margins are freed of the protective sleeve (s). U. S. Patent 5,403,341 to Solar, relates to a stent delivery and deployment assembly which uses retaining sheaths positioned about opposite ends of the compressed stent. The retaining sheaths of Solar are adapted to tear under pressure as the stent is radially expanded, thus releasing the stent from engagement with the sheaths.
U. S. Patent No. 5,108,416 to Ryan et al., describes a stent introducer system which uses one or two flexible end caps and an annular socket surrounding the balloon to position the stent during introduction to the deployment site.

This invention provides an improvement over the cited art, by selectively coating or otherwise lubricating, the sleeve subsequent to its extrusion yet prior to heat curing. This is in contrast to prior methods of lubricating the sleeve, such as by incorporating a lubricant additive within the polymeric composition of the sleeve, such as described in U. S. Patent Application No. 09/273,520., In addition, the present invention avoids the use of collars, rings or other devices used to secure the sleeves to the catheter by bonding an end of a sleeve to the catheter directly.

US 5,944,726 A discloses a stent delivery system including a catheter, balloon and sleeves and a silicone fluid between the balloon and the sleeves.

US 5,295,978 A discloses biocompatible polymeric complexes having utility in medical applications, some of which can render the surface lubricious when exposed to aqueous fluids.

EP-A-688 545 discloses a stent delivery system with a non-silicone based lubricant on the outside and/or inside of the catheter sheath.

### BRIEF SUMMARY OF THE INVENTION

In the present invention, the sleeves are positioned around the catheter with one end portion of each sleeve connected thereto. The other end of each sleeve overlaps an opposite end portion of the stent to hold it in place on the catheter in a contracted condition. The sleeves are elastomeric in nature so as to stretch and release the stent when it is expanded for delivery. A viscous jelly-like lubricant material is provided on the inside surface of the sleeve between the sleeve and the balloon on the catheter, the outside surface of the sleeve, or on both. In a preferred embodiment, a fluid or dry lubricant is coated onto the sleeve material after it has been extruded. Once the lubricant is applied the coated material is cured. The curing process leads to a gelling of the lubricant resulting in the presently desired lubricious yet viscous lubricant material which offers resistance to flow, herein termed a "lubricious gel" coating.

Dry lubricants also find utility in the present invention, and may used alone, or in combination with a fluid lubricant.

Examples of dry lubricants useful herein include those described as solid lamellar form lubricants such as graphite and modified tungsten disulfides such as those sold under the tradename of DICRONITE® available from Dicronite Dry Lube.

Another dry lubricant which may be applied using vapor deposition techniques is sold under the tradename of PARYLENE®. These materials are high molecular weight hydrocarbon materials available from Advanced Coating in Rancho Cucamonga, CA. PARYLENE® materials are referred to as di-para-xylylene (dimers) materials and are available in several forms including the following:

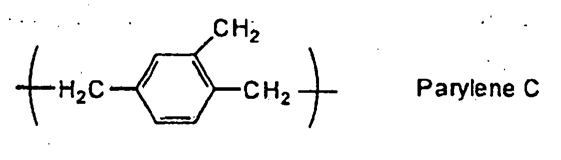

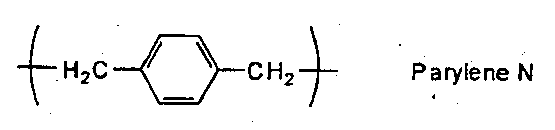

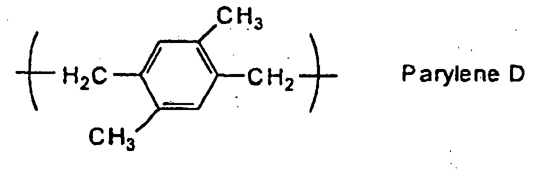

In alternative embodiments of the invention the outside surface of the respective sleeves may be coated as well as the inner surface, or only specific portions of the outer and/or inner surfaces of the sleeves are coated.

In another specific embodiment of the invention, the outer surface of the sleeves are coated with a lubricious hydrophilic coating.

Non-crosslinkable hydrophilic lubricants which may be used with the present invention include: polyalkylene glycols; alkoxy polyalkylene; copolymers of methyl vinyl ether and maleic acid; pyrrolidones including poly(vinylpyrrolidone); acryl amides including poly(N-alkylacrylamide); poly(acrylic acid); poly(vinyl alcohol); poly(ethyleneimine); poly amides; methyl cellulose; hepartin; dextran; modified dextran; chondroitin sulfate; lecithin, etc. These polymers typically contain a hydrophilic group such as: -OH, -CONH₂, -COOH, -NH₂, -COO-, -SO₃, -NR₃⁺, etc.

Some examples of crosslinkable hydrophilic lubricants which may alternatively be utilized with the present invention include: esterified polymers, salts, amides, anhydrides, halides, ethers, hydrolyzates, acetals, formals, alkylols, quaternary polymers, diazos, hydrazides, sulfonates, nitrates and ion complexes.

### BRIEF DESCRIPTION OF THE SEVERAL VIEWS OF THE DRAWINGS

A detailed description of the invention is hereafter described with specific reference being made to the drawings in which:
Fig. 1 is a schematic sectional side view of an embodiment of the inventive stent delivery system wherein the sleeves are coated with lubricious gel on their inside and outside surfaces;
Fig. 2 is a similar view showing the embodiment of the stent delivery system shown in Fig. 1 when the balloon has been inflated to the inflated state.
Fig. 3 is a similar view showing an embodiment of the stent delivery system wherein the sleeves are coated with lubricious gel on only their inside surfaces;
Fig. 4 is a similar view showing an embodiment of the stent delivery system wherein only a portion of the inside surface of the sleeves is coated with lubricious gel;
Fig. 5 is a similar view showing an embodiment of the stent delivery system wherein the sleeves are extruded from different polymer compositions which have then been bonded together;
Fig. 6 is a similar view showing an embodiment of a stent delivery sleeve having a continuous tri-layer construction;
Fig. 7 is a similar view showing an embodiment of a stent delivery sleeve having a partial tri-layer construction; and
Fig. 8 is illustrative of an embodiment of the stent delivery system wherein the outer surface of the sleeves is coated with a lubricious coating material.

### DETAILED DESCRIPTION OF THE INVENTION

While this invention may be embodied in many different forms, there are shown in the drawings and described in detail herein specific preferred embodiments of the invention. The present disclosure is an exemplification of the principles of the invention and is not intended to limit the invention to the particular embodiments illustrated.

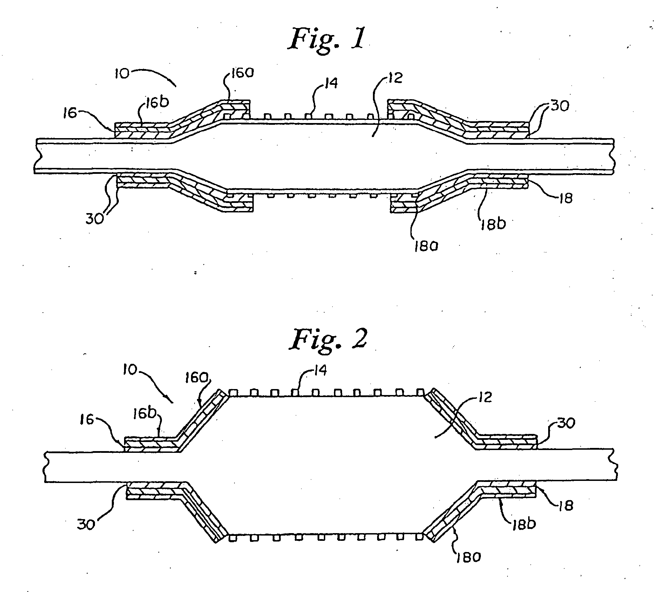

Fig. 1 shows an embodiment of the present invention wherein a catheter generally designated 10 has an expandable portion or balloon 12. The expandable portion may be an inherent part of the catheter, as shown, or alternatively may be a separate balloon which is affixed to the catheter in any of the manners which may be known to one of ordinary skill in the art. Disposed about balloon 12 is a stent 14 as shown. Stent 14 may be any stent type capable of being delivered by a stent delivery catheter, such stents may be self-expanding or balloon expandable.

Attached to the catheter 10 are a pair of sleeves 16,18. The sleeves each include a first portion 16a, 18a. When the balloon 12 is in the non-inflated state first sleeve portions 16a, 18a overlay the ends of balloon 12 as well as the ends of stent 14 as shown. Sleeves 16 and 18 also include respective second portions 16b and 18b.

Regardless of the state of the balloon 12, non-inflated or inflated, second sleeve portions 16b, 18b are fixedly attached to catheter 10. The second sleeve portions may be attached to the catheter utilizing any method of attachment known. Such methods of attachment may include, but are not limited to: bonding or welding the sleeves to the catheter surface, applying an adhesive between the catheter and sleeve surface, or employing a mechanical attachment device such as a retaining ring or collar as is well known in the art. Preferably, the sleeves each have a thickness within the range of 25,4 µm to 152,4 µm (0.0010 to 0.0060 inches).

It is known in the art that in many stent delivery systems a silicone based lubricant is applied to stent retaining sleeves or socks after the delivery system is constructed and the sleeves are in place. However, it is also known that liquid silicone based lubricants applied in this manner tend to be drawn to or wick over the various surfaces of the stent. This is undesirable as the silicone based lubricant may then be introduced into the vessel wall of the patient when the stent is delivered into a body lumen, resulting in potential inflammation and restenosis. In addition, because the stent tends to wick the silicone based lubricant on both its upper and lower surfaces, the stent itself has reduced contact with the balloon surface. As a result it is more difficult to secure the stent to the balloon. The affected stent causes increased crimping pressure which results in crimping processes which may be prone to more readily cause the stent to rupture the balloon.

In this embodiment the present invention avoids the problems mentioned above, by placing a coating of lubricious gel 30 on the interior and exterior surfaces of sleeves 16 and 18 after the sleeve material has been extruded. In order to achieve the desired gel consistency, a suitable fluid lubricant is added to the sleeve material and then heat cured. Heat curing the fluid lubricant coating allows the coating to gel as is desired.

The resulting lubricious coating has a gel-like state as defined herein above, and does not wick or have a tendency to migrate off of the sleeve as prior liquid silicone based lubricant coatings does. The lubricious coating may be comprised of either hydrophobic or hydrophilic compounds.

For illustrative purposes, lubricious coating 30 is shown in the various drawings with a highly exaggerated thickness. When applied after extrusion, lubricious coating 30 is preferably a thin layer. After the heat curing process is complete, preferably, the gelled lubricious coating 30 is a layer less than 2,54 µm (0.0001 inches) in thickness. The physical characteristics of the gelled lubricious coating are such that migration of the coating onto the surfaces of the stent is prevented, unlike the prior slip coatings described above. By preventing the coating from moving on to the stent, the present stent deployment system has reduced parameters for crimping the stent to the balloon, which provides for a crimping process which is much more balloon friendly.

Lubricious coating 30 assists in deployment of stent 14 by allowing the ends of balloon 12 and stent 14 to slide more readily away from the sleeves when balloon 12 is inflated, as seen in Fig. 2. Once the ends of stent 14 are no longer overlaid by sleeves 16 and 18 the stent is allowed to fully expand.

In one preferred embodiment of the present invention, as shown in Fig. 1, sleeves 16 and 18 may have a lubricious coating on both their inside surfaces as well as their outside surfaces. A lubricious coating on the outside surfaces may provide improved trackability and movement of the catheter in a body lumen. In an alternative embodiment of the invention it may be desirable to include a sheath around the region of the catheter where the stent is mounted. A lubricious coating on the outside surface of the sleeves may assist in the retraction of such a catheter sheath by reducing the amount of resistance the sheath must overcome in order to be retracted from the stent mounting region. In addition the outside lubricious coating, may reduce the likelihood of the sheath hooking or pulling on the sleeves or stent as it is pulled back.

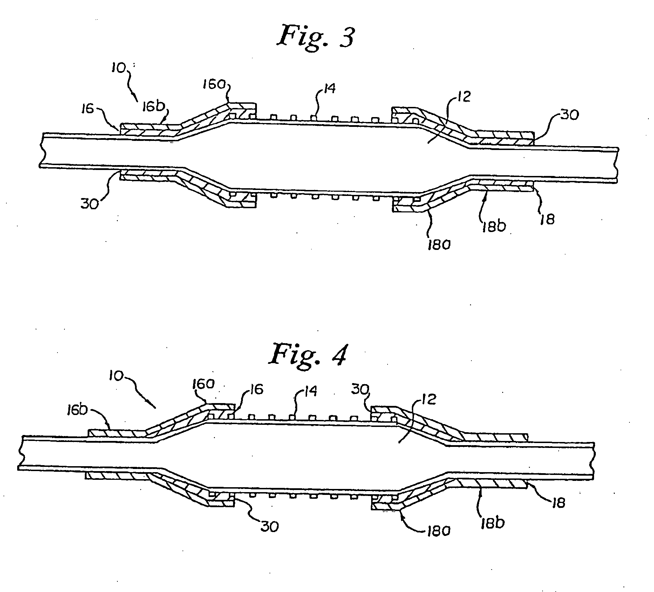

In a further preferred embodiment of the present invention it may be desirable to coat only the inside surfaces of the sleeves. As shown in Fig. 3, only the inside surface of sleeves 16 and 18 are coated with lubricious coating 30.

Because different lubricious coating types may have diverse characteristics, some lubricious coatings may interfere with the attachment of the sleeves to the catheter. In such an instance, it may be desirable or necessary to coat only specific portions of the sleeves. More specifically, in order to ensure proper securement of second sleeve portions 16b and 18b to catheter 10 it may be desirable or necessary to avoid coating the second sleeve portions, as shown in Fig. 4. However, the benefits provided by lubricious coating 30 are substantially maintained in this instance by coating only the inside surface of first sleeve portions 16a and 16b, thereby ensuring that the ends of the stent and balloon may be readily withdrawn from under the sleeves when the balloon is inflated.

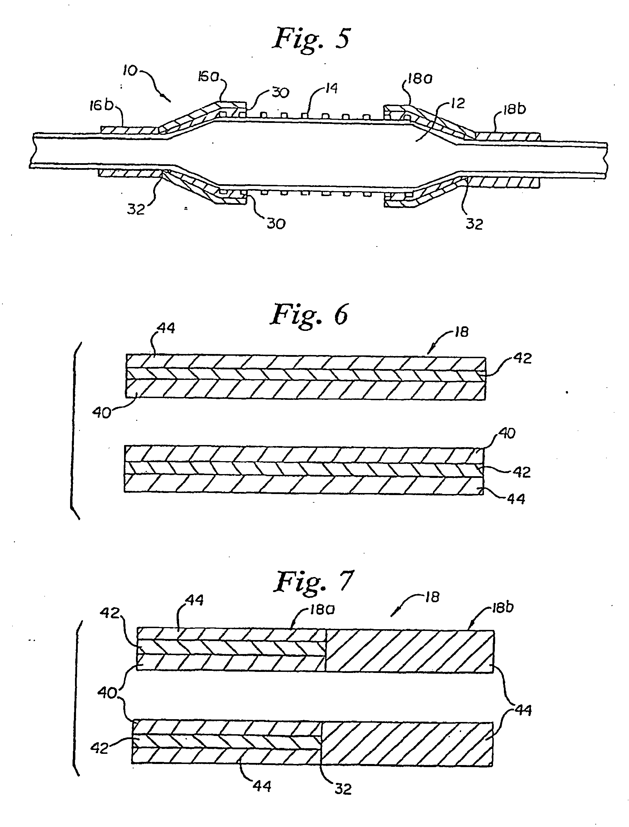

Because of various manufacturing limitations inherent in the production of elastomeric polymer sleeves of the type described and preferably used herein, it is often more desirable to extrude and shape the polymer material into a tube which is to be used in the manufacture of the sleeve, then to separate the portion of the tube which will overlie the ends of the stent and balloon and separately coat these sections i.e., 16a and 18a. After the appropriate sections are coated they may be heat cured and then bonded, welded or otherwise attached to the uncoated sections 16b and 18b which will be connected to the catheter. The embodiment shown in Fig. 5 shows such the stent delivery system with such bonded sleeves. First sleeve sections 16a and 18a have lubricous coating 30 applied to their inside surfaces. They are then connected to the second sleeve sections 16b, 18b with a weld 32. Weld 32 may be a lap weld, a butt weld, an adhesive or any other means of connection which may be known to one of ordinary skill in the art.

Because stent retaining sleeves may be composed from materials which may be unsuitable for placing an effective layer of lubricious material upon, in another embodiment of the present invention the sleeves may have a tri-layer construction such as shown in Figs. 6 and 7. Where the sleeves have a tri-layer construction the sleeves may be comprised of an inner layer 40 which is an inherently lubricous polymer such as polytetrafluoroethylene (PTFE), high density polyethylene (HDPE), acetal resins such as those available from the Dupont corporation such as DELTRIN^{®} or other suitable polymer types.

In Figs. 6 and 7, in order to show two potential embodiments of sleeves which may include the tri-layer construction described above, the sleeves are shown in an exaggerated scale. Furthermore, respective Figs. 6 and 7 each show only a single sleeve 18, sleeve 16 is a left-handed mirror image of sleeve 18 as shown. In the embodiment shown in Fig. 6, inner layer 40 may extend through out the length of a sleeve 18 or in an alternative embodiment shown in Fig. 7, may be confined to only a portion of the sleeve such as the first sleeve portions 16a and 18a. Opposite the inner layer 40 is outer layer 44. Outer layer 44 is composed of any polymer material which can be used in any of the embodiments of the present invention already described herein, preferably having elastomer properties as well as heat shrinkable properties. The lubricious inner layer 40 and the outer polymer layer 44 are joined by an intermediate layer 42. The intermediate layer or third is composed of material which is characterized as being capable of bonding to the inner lubricous polymer material on one surface, and the outer sleeve polymer material on the other. Preferably, the intermediate layer is composed of PLEXAR^{®} 380, thermoplastic polymers including polypropylene, polyurethane or other similar materials.

In the embodiment shown in Figs. 7, it may also be more desirable to bond first sleeve portion 18a, to the second sleeve portion 18b, with a weld 32 or other method of attachment as described as described in relation to Fig. 5 above.

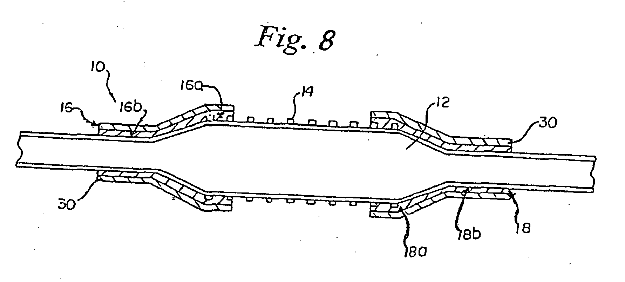

In yet another preferred embodiment of the present invention it may be desirable to coat only the outer surfaces of the sleeves, although as noted above, both the inner and outer surfaces may be coated, or coating only the inner surface is also an option. As shown in Fig. 8, only the outer surface of sleeves 16 and 18 are coated with lubricious coating 30 to improve the lubricity.

Preferably, when the lubricious coating 30 is provided on the outer surface of the sleeves 16 and 18, it is a hydrophilic coating.

The sleeves 16 and 18 are preferably coated without coating the stent or balloon structures themselves. The coating may be applied either by dipping or by brushing. Dipping, however, typically involves coating both the inner and outer surfaces of the sleeves 16 and 18 as shown in Fig. 1. The coating typically requires drying or curing time which time may be decreased by the addition of a heating source. The hydrophilic coating may be applied at a thickness of about 0.2 to about 20 µm.

There are many hydrophilic compounds that may be utilized in the present invention. The water soluble lubricants useful herein include polyalkylene glycols, alkoxy polyalkylene glycols, homopolymers and copolymers of (meth) acrylic acid, copolymers of methylvinyl ether and maleic acid, poly(vinylpyrrolidone) homopolymers, copolymers of vinyl pyrrolidone, poly(N-alkylacrylamide), poly(vinyl alcohol), poly(ethyleneimine), polyamides, methyl cellulose, carboxymethylcellulose, polyvinylsulfonic acid, heparin, dextran, modified dextran, chondroitin sulphate and lecithin. The polymers are typically chain-structured, non-crosslinked and water soluble having a hydrophilic group such as -OH, -CONH₂, -COOH, -NH₂, -COO-, -SO₃, -NR₃⁺ and so forth where R is alkyl or hydrogen. These water soluble polymers are typically chain-structured, non-crosslinked polymers having a hydrophilic group such as -OH, - CONH₂, -COOH, -NH₂, -COO-, SO₃, AND NR₃⁺, where R is alkyl or hydrogen.

Natural hydrophilic polymers may also be utilized such as carboxymethyl cellulose, methyl cellulose, hydroxyethyl cellulose and hydroxypropyl cellulose, heparin, dextran, modified dextran and chondroitin sulphate.

Synthetic hydrophilic polymers include the polyalkylene glycols and polyoxyalkylene glycols such as polyethylene oxide, polyethylene oxide/polypropylene oxide copolymers and methoxypolyethylene oxide; copolymers of maleic anhydride including methyl vinyl ether-maleic anhydride copolymers; pyrrolidones including poly(vinylpyrrolidone); acryl amides including poly(N-alkylacrylamide); poly(acrylic acid); poly(carboxylic acids); poly(vinyl alcohol); poly(ethyleneimine); water soluble nylons; polyurethanes; and so forth.

Derivatives of any of these polymers may be utilized providing that enough of the basic structure of the polymers above that provides water sensitivity, solubility or dispersibility is retained allowing the polymer to uptake enough water to swell or partially dissolve enough upon exposure to moisture to provide lubricity in such a way to reduce frictional forces between the surface it is coated on and another surface such as tissue, metal or polymeric surfaces. Water insoluble derivatives may be employed as long as they have the freedom in the molecular chain and can be hydrated. Examples include esterified polymers, salts, amides, anhydrides, halides, ethers, hydrolyzates, acetals, formals, alkylols, quaternary polymers, diazos, hydrazides, sulfonates, nitrates, and ion complexes which are obtained by condensation, addition, substitution, oxidation, or reduction reactions of the above-mentioned water soluble polymers. Also used are polymers crosslinked with substances having more than one reactive functional group such as diazonium, azide, isocyanate, acid chloride, acid anhydride, imino carbonate, amino, carboxyl, epoxy, hydroxyl, and aldehyde groups.

A particular type of hydrophilic polymer useful herein include those polymers that have the ability to dissolve or swell in an aqueous environment, often referred to as "hydrogels." These polymers are capable of manifesting lubricity while in a wet state, and when hydrated, exhibit low frictional forces in humoral fluids such as saliva, digestive fluids and blood, as well as in saline solution and water.

Such hydrogel compounds include polyethylene oxides (optionally linked to the substrate surface by interpenetrating network, IPN, with poly(meth)acrylate polymers or copolymers; copolymers of maleic anhydride; (meth)acrylamide polymers and copolymers; (meth)acrylic acid copolymers; poly(vinyl pyrrolidone) and blends or interpolymers with polyurethanes; and polysaccharides.

Other polymeric materials which hydrogels may comprise include polyalkylene glycols, alkoxy polyalkylene glycols, copolymers of methylvinyl ether and maleic acid, poly(N-alkylacrylamide), poly(acrylic acid), poly(vinyl alcohol), poly(ethyleneimine), polyamides, methyl cellulose, carboxymethyl cellulose, polyvinyl sulfonic acid, heparin, dextran, modified dextran and chondroitin sulphate.

In a specific embodiment, a hydrogel of polyethylene oxide may be captured in an interpenetrating crosslinked acrylic polymer network by polymerizing a mixture of an acrylic monomer composition comprising a monomer having plural (meth)acrylate groups and polyethylene oxide, thereby providing a hydrogel coating.

Copolymers with vinyl groups, acrylic acid, methacrylic acid, fumaric acid, maleic acid, maleic anhydride, diene compounds, or other polymerizable ethylenically unsaturated compounds are another particular group of polymers which find utility herein. The acids may be optionally be neutralized.

Examples of copolymers based on maleic anhydride include poly(ethylene-maleic anhydride) sold by Aldrich Chemical Co. or maleic anhydridemethyl vinyl ether copolymers such as Gantrez^{®} AN 169 sold by G.A.F. Corporation.

Other specific hydrophilic coatings which find particular utility herein include the polyethylene oxides, polyacrylic acids and polyvinylpyrrolidones.

The hydrophilic polymers of the present invention may be utilized in any combination to more narrowly tailor the resultant composition to the application. Some of the hydrophilic polymers of the present invention exhibit less flexibility than others. For instance, the flexibility of the hydrogels found in the previous paragraph above, may be improved by the addition of polyethylene oxide/polypropylene oxide copolymers, especially block copolymers, poly(vinyl pyrrolidone), polyvinyl alcohol, and so forth.

The present invention also contemplates the use of slip additives or antiblock agents to the hydrophilic coatings of the present invention, particularly in those embodiments in which the outer surface of the sleeves are coated with a lubricious hydrophilic coating.

The coating compositions of the present invention may be coated out of a solvent or a cosolvent mixture using any conventional coating techniques such as dipping, spraying, brushing, and so forth.

While these hydrophilic coatings have been discussed in relation to an embodiment directed more toward coating the outer surface of sleeves, they may also be used to coat the inner surface of the sleeves, or both.

Useful solvents include alcohols, aliphatic hydrocarbons, aromatic hydrocarbons, chlorinated solvents, esters, glycols, glycol ethers, ketones, and so forth. Polar solvents include alcohols, glycols, water and so forth. Specific examples include ethanol, methanol, isopropanol, stearyl alcohol, ethylene glycol, propylene glycol, glycerin, water and so forth. Non-polar solvents include aliphatic hydrocarbons such as heptane and hexane; aromatic hydrocarbons such as toluene and xylene; chlorinated hydrocarbons such as perchloroethylene, methylene chloride, chloroform, carbon tetrachloride, 1,1,1-trichloroethane; fluorocarbons; mineral spirits and so forth.

In the case of hydrophilic coatings, the preferable solvents are more polar and preferably include the alcohols such as isopropyl alcohol or isopropanol and water and mixtures thereof.

This completes the description of the preferred and alternate embodiments of the invention. Those skilled in the art may recognize other equivalents to the specific embodiment described herein which equivalents are intended to be encompassed by the claims attached hereto.

## Claims

1. A stent delivery system comprising a stent delivery catheter (10) which is equipped with at least one stent retaining sleeve (16, 18), the at least one stent retaining sleeve (16, 18) having an inside surface and an outside surface, said stent retaining sleeve (16, 18) having a lubricious coating on at least a portion of at least one of said inside surface and said outside surface **characterized in that** the lubricious coating is a non-silicone based lubricant, wherein said lubricant has been heat cured to a gel (30).

2. The stent delivery system of Claim 1 wherein the at least one stent retaining sleeve (16, 18) is formed of a material which is first extruded and then cured, the material being.at least partially coated with a lubricious substance after the material is extruded but before the material is heat cured.

3. The stent delivery system of Claim 1 wherein at least one stent retaining sleeve (16, 18) is comprised of an extruded polymeric material.

4. The stent delivery system of Claim 1 wherein at least a portion of the outside surface of said sleeve (16, 18) is coated with a lubricious coating.

5. The stent delivery system of Claim 1 wherein at least a portion of the inside surface of said sleeve (16, 18) is coated with a lubricious coating.

6. The stent delivery system of Claim 1 wherein said lubricious coating is hydrophilic.

7. The stent delivery system of Claim 1 wherein said lubricious coating comprises at least one compound selected from hydrogels, copolymers of polyalkylene oxides, homopolymers or copolymers of vinyl pyrrolidone, copolymers of at least one polymerizable ethylenically unsaturated compound, and mixtures thereof.

8. The stent delivery system of Claim 7 wherein said hydrogel is a hydrogel of polyethylene oxide captured in an interpenetrating crosslinked acrylic polymer network by polymerizing a mixture of an acrylic monomer composition comprising a monomer having plural (meth) acrylate groups and polyethylene oxide, thereby providing a hydrogel coating.

9. The stent delivery system of Claim 1 wherein said lubricious coating comprises at least onepolycarboxylic acid.

10. The stent delivery system of Claim 7 wherein said copolymer is a copolymer of at least one selected from maleic acid, fumaric acid, (meth) acrylic acid, maleic anhydride, and mixtures thereof.

11. The stent delivery system of Claim 10 wherein said maleic anhydride copolymer is selected from poly (ethylene-maleic anhydride) copolymers and maleic anhydridemethyl vinyl ether copolymers.

12. The stent delivery system of Claim 1 wherein said lubricious coating is comprised of a polymer system which requires curing or drying.

13. The stent delivery system of Claim 1 wherein said lubricious coating comprises a dry lubricant material.

14. The stent delivery system of Claim 1 wherein the lubricious coating is a lubricious gel (30).

15. The stent delivery system of Claim 1 wherein the inside surface is further characterized as having a first portion and a second portion, the first portion being the portion of the inside surface which overlays the stent on the expandable portion of the catheter, the second portion being the portion of the inside surface which is attached to the catheter, at least the first portion being coated with a lubricious gel (30).

16. The stent delivery system of Claim 1 wherein the at least one sleeve (16, 18) is further characterized as having a first portion (16a, 18a) and a second portion (16b, 18b), the first portion (16a, 18a) being the portion of the at least one sleeve (16, 18) which overlays the stent on the expandable portion of the catheter (10), the second portion (16b, 18b) being the portion of the at least one sleeve (16, 18) which is attached to the catheter (10), the first portion (16a, 18a) being composed of an extruded polymer in combination with the lubricious gel (30), there being no lubricious gel (30) in the composition of the second portion (16b, 18b).

17. The stent delivery system of Claim 16 wherein the first portion (16a, 18a) and the second portion (16b, 18b) are bonded together by a method selected from adhesively, laser bonding and welding.

18. The stent delivery system of Claim 1 wherein said lubricant comprises a crosslinkable lubricant, the crosslinkable lubricant comprises at least one member selected from the group consisting of esterified polymers, salts, amides, anhydrides, halides, ethers, hydrolyzates, acetals, formals, alkylols, quaternary polymers, diazos, hydrazides, sulfonates, nitrates, ion complexes.

## Patentansprüche

1. Stentzuführsystem, aufweisend einen Stentzuführkatheter (10), der mit mindestens einer Stenthaltehülse (16, 18) ausgestattet ist, wobei die mindestens eine Stenthaltehülse (16, 18) eine Innenfläche und eine Außenfläche aufweist, wobei die Stenthaltehülse (16, 18) eine Gleitbeschichtung an mindestens einem Abschnitt der Innenfläche und/oder Außenfläche aufweist, **dadurch gekennzeichnet, dass** die Gleitbeschichtung ein nicht auf Silikon basierendes Gleitmittel ist, wobei das Gleitmittel durch Wärme zu einem Gel (30) ausgehärtet wird.

2. Stentzuführsystem nach Anspruch 1, wobei die mindestens eine Stenthaltehülse (16, 18) aus einem Material gebildet ist, das erst extrudiert und anschließend ausgehärtet wird, wobei das Material nach dem Extrudieren aber vor dem Wärmehärten zumindest teilweise mit einer Gleitsubstanz beschichtet wird.

3. Stentzuführsystem nach Anspruch 1, wobei mindestens eine Stenthaltehülse (16, 18) aus einem extrudierten Polymerwerkstoff besteht.

4. Stentzuführsystem nach Anspruch 1, wobei mindestens ein Abschnitt der Außenfläche der Hülse (16, 18) mit einer Gleitbeschichtung beschichtet ist.

5. Stentzuführsystem nach Anspruch 1, wobei mindestens ein Abschnitt der Innenfläche der Hülse (16, 18) mit einer Gleitbeschichtung beschichtet ist.

6. Stentzuführsystem nach Anspruch 1, wobei die Gleitbeschichtung hydrophil ist.

7. Stentzuführsystem nach Anspruch 1, wobei die Gleitbeschichtung mindestens eine Verbindung aufweist, die aus Hydrogelen, Copolymeren von Polyalkylenoxiden, Homopolymeren oder Copolymeren von Vinylpyrrolidon, Copolymeren von mindestens einer polymersisierbaren ethylenisch ungesättigten Verbindung und Gemischen daraus besteht.

8. Stentzuführsystem nach Anspruch 7, wobei das Hydrogel ein Hydrogel aus Polyethylenoxid ist, das in einem vernetzten interpenetrierenden Acrylpolymer-Netzwerk, gefangen ist, in dem ein Gemisch aus einer Acrylmonomerzusammensetzung, die ein Monomer umfasst, das mehrere Methacrylatgruppen aufweist, und Polyethylenoxid polymerisiert wird, wodurch eine Hydrogelbeschichtung geschaffen wird.

9. Stentzuführsystem nach Anspruch 1, wobei die Gleitbeschichtung mindestens eine Polycarbonsäure aufweist.

10. Stentzuführsystem nach Anspruch 7, wobei das Copolymer ein Copolymer von mindestens einem Element ist, das aus Maleinsäure, Fumarsäure, (Meth)Acrylsäure, Maleinsäureanhydrid und Gemischen daraus ausgewählt ist.

11. Stentzuführsystem nach Anspruch 10, wobei das Maleinsäureanhydrid-Copolymer aus Polyethylen-Maleinsäureanhydrid-Copolymeren und Maleinsäureanhydrid-Methylvinylether-Copolymeren ausgewählt ist.

12. Stentzuführsystem nach Anspruch 1, wobei die Gleitbeschichtung aus einem Polymersystem besteht, das eine Härtung oder Trocknung erfordert.

13. Stentzuführsystem nach Anspruch 1, wobei die Gleitbeschichtung ein trockenes Gleitmaterial umfasst.

14. Stentzuführsystem nach Anspruch 1, wobei die Gleitbeschichtung ein Gleitgel (30) ist.

15. Stentzuführsystem nach Anspruch 1, wobei die Innenfläche ferner **dadurch gekennzeichnet ist, dass** sie einen ersten Abschnitt und einen zweiten Abschnitt aufweist, wobei der erste Abschnitt der Abschnitt der Innenfläche ist, der auf dem dehnbaren Abschnitt des Katheters über dem Stent liegt, wobei der zweite Abschnitt der Abschnitt der Innenfläche ist, der am Katheter befestigt ist, wobei zumindest der erste Abschnitt mit dem Gleitmaterial beschichtet ist.

16. Stentzuführsystem nach Anspruch 1, wobei die mindestens eine Hülse (16, 18) ferner **dadurch gekennzeichnet ist, dass** sie einen ersten Abschnitt (16a, 18a) und einen zweiten Abschnitt (16b, 18b) aufweist, wobei der erste Abschnitt (16a, 18a) der Abschnitt der mindestens einen Hülse (16, 18) ist, der auf dem dehnbaren Abschnitt des Katheters (10) über dem Stent liegt, wobei der zweite Abschnitt (16b, 18b) der Abschnitt der mindestens einen Hülse (16, 18) ist, der am Katheter (10) befestigt ist, wobei der erste Abschnitt (16a, 18a) aus einem extrudierten Polymer in Verbindung mit dem Gleitgel (30) besteht, wobei kein Gleitgel (30) in der Zusammensetzung des zweiten Abschnitts (16b, 18b) vorhanden ist.

17. Stentzuführsystem nach Anspruch 16, wobei der erste Abschnitt (16a, 18a) und der zweite Abschnitt (16b, 18b) durch ein Verfahren miteinander verbunden sind, das aus Kleben, Verbinden mit einem Laser und Schweißen ausgewählt ist.

18. Stentzuführsystem nach Anspruch 1, wobei das Gleitmittel ein vernetzbares Gleitmittel aufweist, wobei das vernetzbare Gleitmittel mindestens ein Element aus der Gruppe bestehend aus veresterten Polymeren, Salzen, Amiden, Anhydriden, Halogeniden, Ethern, Hydrolysaten, Acetalen, Formaldehydacetale (engl. formals), Alkanole, quartären Polymeren, Diazoverbindungen (engl. diazos), Hydraziden, Sulfonaten, Nitraten, Ionenkomplexen, aufweist.

## Revendications

1. Système d'insertion de stent comprenant un cathéter de pose de stent (10) muni d'au moins un manchon de rétention de stent (16, 18), le au moins un manchon de rétention de stent (16, 18) comportant une surface intérieure et une surface extérieure, ledit manchon de rétention de stent (16, 18) ayant un revêtement lubrifiant sur au moins une partie d'au moins une surface parmi ladite surface intérieure et ladite surface extérieure, **caractérisé en ce que** le revêtement lubrifiant est un lubrifiant non-siliconée, dans lequel ledit lubrifiant a été thermodurci pour former un gel (30).

2. Système d'insertion de stent selon la revendication 1, dans lequel le au moins un manchon de rétention de stent (16, 18) est constitué d'un matériau qui est d'abord extrudé et ensuite durci, le matériau étant au moins partiellement revêtu avec une substance lubrifiante après que le matériau est extrudé mais avant que le matériau soit thermodurci.

3. Système d'insertion de stent selon la revendication 1, dans lequel au moins un manchon de rétention de stent (16, 18) est constitué d'un matériau polymère extrudé.

4. Système d'insertion de stent selon la revendication 1, dans lequel au moins une partie de la surface extérieure dudit manchon (16, 18) est enduite d'un revêtement lubrifiant.

5. Système d'insertion de stent selon la revendication 1, dans lequel au moins une partie de la surface intérieure dudit manchon (16, 18) est enduite d'un revêtement lubrifiant.

6. Système d'insertion de stent selon la revendication 1, dans lequel ledit revêtement lubrifiant est hydrophile.

7. Système d'insertion de stent selon la revendication 1, dans lequel ledit revêtement lubrifiant comprend au moins un composé sélectionné parmi les hydrogels, les copolymères de polyoxydes d'alkylène, les homopolymères ou copolymères de vinyl pyrrolidone, les copolymères d'au moins un composé insaturé polymérisable sur le plan éthylénique, et des mélanges de ceux-ci.

8. Système d'insertion de stent selon la revendication 7, dans lequel ledit hydrogel est un hydrogel de polyoxyde d'éthylène capturé dans un réseau de polymères d'acrylique réticules s'interpénétrant en polymérisant un mélange d'une composition de monomère d'acrylique comprenant un monomère comportant plusieurs groupes méthacrylate et du polyoxyde d'éthylène, produisant ainsi un revêtement d'hydrogel.

9. Système d'insertion de stent selon la revendication 1, dans lequel ledit revêtement lubrifiant comprend au moins un acide polycarboxylique.

10. Système d'insertion de stent selon la revendication 7, dans lequel ledit copolymère est un copolymère d'au moins un élément sélectionné parmi l'acide maléique, l'acide fumarique, l'acide méthacrylique, l'anhydride maléique, et les mélanges de ceux-ci.

11. Système d'insertion de stent selon la revendication 10, dans lequel ledit copolymère d'anhydride maléique est sélectionné parmi les copolymères de polyéthylène et d'anhydride maléique et les copolymères d'anhydridmethyl maléique et d'éther vinylique.

12. Système d'insertion de stent selon la revendication 1, dans lequel ledit revêtement lubrifiant est constitué d'un système polymérique nécessitant le durcissement ou le séchage.

13. Système d'insertion de stent selon la revendication 1, dans lequel ledit revêtement lubrifiant comprend un matériau lubrifiant sec.

14. Système d'insertion de stent selon la revendication 1, dans lequel le revêtement lubrifiant est un gel lubrifiant (30).

15. Système d'insertion de stent selon la revendication 1, dans lequel la surface intérieure est en outre **caractérisée par le fait qu'**elle comporte une première partie et une seconde partie, la première partie étant la partie de la surface intérieure qui se superpose au stent sur la partie étirable du cathéter, la seconde partie étant la partie de la surface intérieure qui est fixée au cathéter, au moins la première partie étant enduite d'un gel lubrifiant (30).

16. Système d'insertion de stent selon la revendication 1, dans lequel le au moins un manchon (16, 18) est en outre **caractérisé par le fait qu'**il comporte une première partie (16a, 18a) and une second partie (16b, 18b), la première partie (16a, 18a) étant la partie du au moins un manchon (16, 18) qui se superpose au stent sur la partie étirable du cathéter (10), la second partie (16b, 18b) étant la partie du au moins un manchon (16, 18) qui est fixée au cathéter (10), la première partie (16a, 18a) étant composée d'un polymère extrudé combiné au gel lubrifiant (30), la composition de la second partie (16b, 18b) ne comportant pas de gel lubrifiant (30).

17. Système d'insertion de stent selon la revendication 16, dans lequel la première partie (16a, 18a) et la seconde partie (16b, 18b) sont liées ensemble à l'aide d'un procédé sélectionné parmi le collage par adhésif, le soudage par laser et le soudage.

18. Système d'insertion de stent selon la revendication 1, dans lequel ledit lubrifiant comprend un lubrifiant réticulable, le lubrifiant réticulable comprenant au moins un élément choisi par le group consistant de polymères estérifiés, de sels, d'amides, d'anhydrides, d'halogénures, d'éthers, d'hydrolysats, d'acétals, de formals, d'alkylols, de polymères quaternaires, de composés diazoïques, d'hydrazides, de sulfonates, de nitrates, de complexes ioniques.
